(19) Europäisches Patentamt
European Patent Office
Office européen des brevets

(11) Publication number: **0 103 552**
**B1**

(12) # EUROPEAN PATENT SPECIFICATION

(45) Date of publication of patent specification: **07.05.86**

(21) Application number: **83850171.6**

(22) Date of filing: **20.06.83**

(88) **Consolidated with 83902142.5/0112364 (European application No./publication No.) by decision dated 18.07.85.**

(51) Int. Cl.$^4$: **C 07 D 473/18,**
C 07 D 473/00,
C 07 D 473/02,
C 07 D 473/30,
C 07 D 473/32, A 61 K 31/52

(54) **Novel derivatives of guanine.**

(30) Priority: **21.06.82 SE 8203856**

(43) Date of publication of application:
**21.03.84 Bulletin 84/12**

(45) Publication of the grant of the patent:
**07.05.86 Bulletin 86/19**

(84) Designated Contracting States:
**AT BE CH DE FR GB IT LI LU NL SE**

(56) References cited:
**EP-A-0 055 239**
**GB-A-1 523 865**
**US-A-4 221 910**
**US-A-4 230 708**

**CHEMICAL ABSTRACTS vol. 80 no 1, 7 January 1974, page 106, column 2, no. 1073q**

**CHEMICAL ABSTRACTS vol. 71, no 13, 19 September 1969, page 454, column 1, no 61344c**

**CHEMICAL ABSTRACTS vol. 82, no 19, 12 May 1975, page 559, column 1, no 125357w**

(73) Proprietor: **Astra Läkemedel Aktiebolag**
**Strängnäsvägen 44**
**S-151 85 Södertälje (SE)**

(72) Inventor: **Eriksson, Bertil Frank Harald**
**Kämpevägen 33**
**S-151 54 Södertälje (SE)**
Inventor: **Gotthammar, Kristina Birgitta**
**Björkholmsvägen 188**
**S-132 00 Saltsjö-Bo (SE)**
Inventor: **Johansson, Karl Nils-Gunnar**
**Bäverstigen 19**
**S-150 23 Enhörna (SE)**

(74) Representative: **Wurm, Bengt Runio et al**
**Patent and Trade Mark Department AB ASTRA**
**S-151 85 Södertälje (SE)**

(56) References cited:
**CHEMICAL ABSTRACTS vol. 56, no 3, 5 February 1962, column 2450-52 (2451g)**

**CHEMICAL ABSTRACTS vol. 89, no. 13, 25 September 1978, page 70, column 1, no 100003j**

**EP 0 103 552 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

The present invention relates to novel derivatives of guanine, methods for their preparation, novel pharmaceutical compositions and said compounds for use in the treatment of virus infections, such as herpes virus infections, which can cause various diseases in an animal or human host, including both common infections and neoplastic diseases, i.e. cancer.

Background of the Invention

The effects of viruses on bodily functions is the end result of changes occurring at the cellular and subcellular levels. The pathogenic changes at the cellular level are different for different combinations of viruses and host cells. While some viruses cause a general destruction (killing) of certain cells, other may transform cells to a neoplastic state.

Important common viral infections are herpes dermatitis (including herpes labialis), herpes keratitis, herpes genitalis, herpes zoster, herpes encephalitis, infectious mononucleosis and cytomegalovirus infections all of which are caused by viruses belonging to the herpes-virus group. Other important viral diseases are influenza A and B which are caused by influenza A and B virus respectively. Another important common viral disease is viral hepatitis and especially hepatitis B virus infections are widely spread. Effective and selective antiviral agents are needed for the treatment of these diseases as well as for other diseases caused by viruses.

Several different viruses of both DNA and RNA type have been shown to cause tumors in animals. The effect of cancerogenic chemicals can on animals result in activation of latent tumor viruses. It is possible that tumor viruses are involved in human tumors. The most likely human cases known today are leucemias, sarcomas, breast carcinomas, Burkitt lymphomas, nasopharyngeal carcinomas and cervical cancers where RNA tumor viruses and herpes viruses are indicated. This makes the search for selective inhibitors of tumorogenic viruses and their functions an important undertaking in the efforts to treat cancer.

Prior Art

The compound 9-(4-hydroxybutyl)-guanine is disclosed in Chem. Pharm. Bull. *17* (1969) 1268—1270 and in Agr. Biol. Chem., *37* (1973) 2037—2043. However, no antiviral or other pharmacological activity has been disclosed for said compound.

US 4 199 574 discloses a broad class of substituted purines of the formula

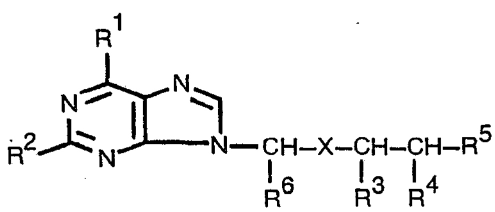

wherein X is oxygen or sulphur; $R^1$ is hydrogen, halogen, hydroxy, alkoxy, azide, thio, alkylthio, amino, alkylamino, or dialkylamino; $R^2$ is hydrogen, halogen, alkylthio, acylamino, amino or azide; $R^3$ is hydrogen, straight or branch chain or cyclic alkyl, hydroxyalkyl, benzyloxyalkyl, or phenyl; $R^4$ is hydrogen, hydroxy or alkyl; $R^5$ is hydrogen, hydroxy, amino, alkyl, hydroxyalkyl, benzyloxy, benzoyloxy, benzoyloxymethyl, sulphamoyloxy, phosphate carboxypropionyloxy, straight chain or cyclic acyloxy having from 1 to 8 carbon atoms e.g., acetoxy or substituted carbamoyl group of formula NHCO—Z wherein Z is alkyl, aryl or aralkyl optionally substituted by one or more of sulphonyl, amino, carbamoyl or halogen; $R^6$ is hydrogen or alkyl, provided that when X is oxygen and $R^2$, $R^3$, $R^4$ and $R^6$ are hydrogen, $R^1$ is not amino or methylamino when $R^5$ is hydrogen or hydroxy. These compounds are asserted to possess antiviral activity against various classes of DNA and RNA viruses. 9-(2-Hydroxyethoxymethyl)guanine and 2-amino-9-(2-hydroxyethoxymethyl)adenine are mentioned as examples of especially active compounds.

Disclosure of Invention

The present invention relates to the novel antiviral compounds of the formula

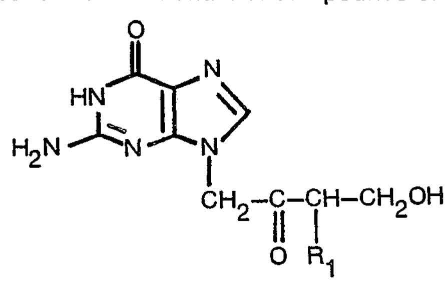

I

wherein $R_1$ is hydrogen, hydroxy or mercapto and physiologically acceptable salts or optical isomers thereof.

It has been found that such compounds exert an antiviral effect and inhibit certain viral functions including tumorogenic functions and the multiplication of viruses.

The invention thus provides compounds, and physiologically acceptable salts thereof, which compounds are useful in therapeutic and/or prophylactic treatment of viral diseases and which may be useful in therapeutic and/or prophylactic treatment of cancer caused by viruses.

An effective selective antiviral agent with acceptable side effects should have a selective inhibiting effect on a specific viral function of the virus to be combated. It is, therefore, one object of the present invention to provide antiviral agents for use in combating virus infections whereby said agents exert a selective inhibiting effect on viral functions but which exert only a negligible inhibiting effect on functions of the host cells.

The invention also relates to novel pharmaceutical compositions containing the antiviral agents.

Although the present invention relates broadly to compounds to be used in a novel method for combating virus infections in animals and man, it will be particularly useful in the treatment of herpes virus infections.

An especially important area of use for the compounds of the present invention is in the treatment of herpes virus infections. Among the herpes viruses may be mentioned Herpes simplex type 1 and 2, varicella (Herpes zoster), virus causing infectious mononucleosis (i.e. Epstein-Barr virus) and cytomegalovirus. Important diseases caused by herpes viruses are herpes dermatitis (including herpes labialis), herpes genitalis, herpes keratitis, herpes encephalitis and herpes zoster.

Another possible area of use for the compounds of the present invention is in the treatment of cancer and tumours, particularly those caused by viruses. This effect may be obtained in different ways, i.e. by inhibiting the transformation of virus-infected cells to a neoplastic state, by inhibiting the spread of viruses from transformed cells to other normal cells and by arresting the growth of virus transformed cells.

A further area of use for the compounds of the present invention is in the inhibition of transformed cells due to the presence in these cells of specific herpes virus enzymes like thymidine kinase.

Possible areas of use for the compounds of the present invention with respect to cancer chemotherapy are treatment of leukaemias, lymphomas including Burkitt lymphomas and Hodgkin's disease, sarcomas, breast carcinoma, nasopharyngeal carcinomas and cervical cancers in which viruses are indicated. Other possible areas of use for the compounds of the present invention with respect to cancer chemotherapy are treatment of multiple myeloma and cancer of the lungs (and bronchus), the stomach, the liver, the colon, the bladder, the lips, the bones, the kidneys, the ovary, the prostate, the pancreas, the skin (melanoma), the rectum, the salivary glands, the mouth, the esophagus, the testis, the brain (and cranial meninges), the thyroid gland, the gallbladder (and ducts), the nose, the larynx, connective tissues, the penis, the vulvas, the vagina, the corpus uteri and the tongue.

The invention furthermore provides

A. The compound of the formula I for use in the treatment of diseases caused by viruses in animals including man, comprising administering to an animal so infected a therapeutially effective amount of a said compound or a physiologically acceptable salt thereof.

B. The compound of the formula I for use in inhibiting the multiplication of virus, in particular herpesviruses, in animals including man, by administering to an animal in need of such treatment a said compound or a physiologically acceptable salt thereof in an amount sufficient for inhibiting said multiplication.

C. The compound of the formula I for use in the treatment of virus-induced neoplastic diseases in animals including man, by inhibiting the growth of cells expressing viral functions, characterized by administering to an animal so infected a therapeutically effective amount of a said compound or a physiologically acceptable salt thereof.

D. The compound of the formula I for use in inhibiting the growth of virus-transformed cells in animals including man, characterized by administering to an animal in need of such treatment a said compound or physiologically acceptable salt thereof in an amount sufficient for inhibiting said growth.

E. The compound of the formula I for use in the treatment of virus-induced neoplastic diseases in animals including man, by inhibiting the multiplication of tumor viruses, characterized by administering to an animal in need of such treatment a said compound or a physiologically acceptable salt thereof in an amount sufficient for inhibiting such multiplication.

F. The compound of the formula I for use in the treatment of neoplastic diseases in animals including man, characterized by administering to an animal a therapeutically effective amount of a said compound or a physiologically acceptable salt thereof.

As stated previously the compounds of the present invention have the formula

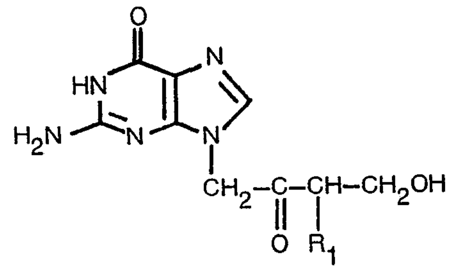

I

wherein $R_1$ is hydrogen, hydroxy or mercapto including physiologically acceptable salts and optical isomers thereof.

The provisos in the definition for the group $R_1$ above mean that only the following three specific compounds, including salts and optical isomers thereof, constitute part of the present invention:

9-(2-oxo-4-hydroxybutyl)guanine
9-(2-oxo-3,4-dihydroxybutyl)guanine
9-(2-oxo-3-mercapto-4-hydroxybutyl)guanine

Two of the compounds of the formula I contain one asymmetric center. Accordingly, they exist in two optical forms, respectively, and all such forms constitute a further aspect of the invention.

Methods of Preparation

The compounds of the invention may be obtained by one of the following methods A—D constituting a further aspect of the invention.

A. Condensing an acyclic side chain, where the functional groups may optionally be protected, to the N-9 position of a guanine derivative, followed by removal of the protecting groups, through one or more chemical reactions.

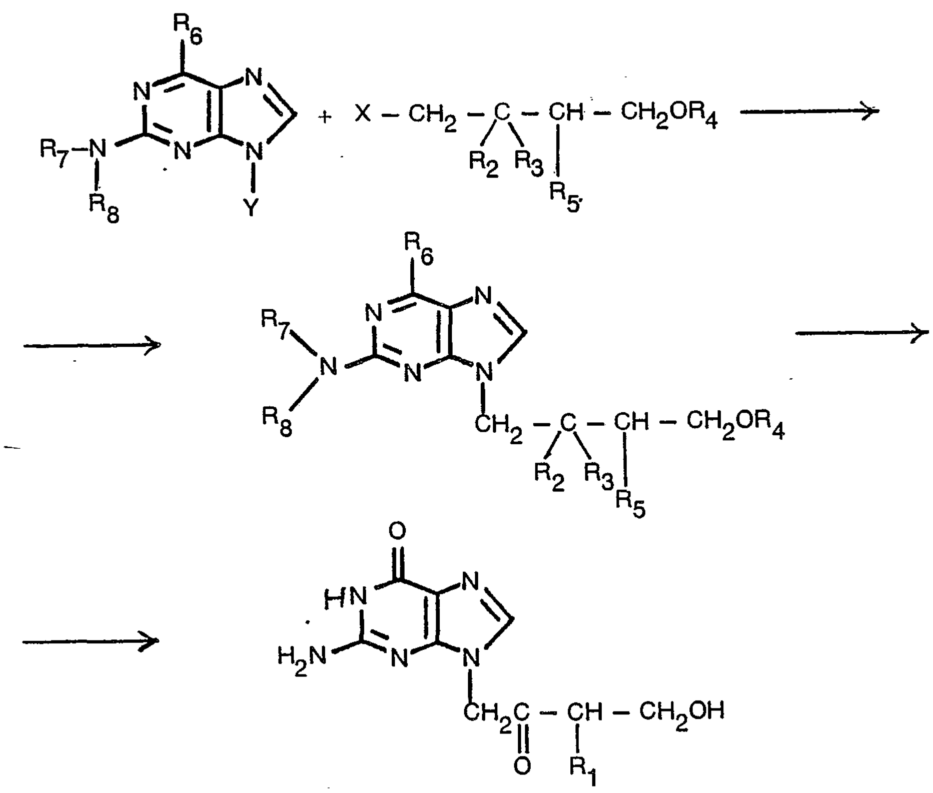

wherein X is a group such as chlorine, bromine, iodine or a group $OSO_2R_9$ where $R_9$ is alkyl containing 1—8 carbon atoms, fluorinated alkyl containing 1—8 carbon atoms, such as trifluoromethyl, aryl or arylalkyl such as benzyl. Y is hydrogen or a quarternary ammonium ion such as for example tetrabutylammonium. $R_2$ and $R_3$ are together double-bonded oxygen, i.e. $CR_2R_3$ is carbonyl, or alkoxy groups, forming open or cyclic ketals. $R_4$ is hydrogen or a hydroxyl protecting group of which a great variety are known to those skilled in the art and are described for example in "Protective Groups in Organic Chemistry" (T. W. Greene, Wiley 1981), "Methoden der Organischen Chemie"(Houben-Weyl) Vol. VI/Ib, or in "Comprehensive Organic

Chemistry" (D. H. R. Barton and W. D. Ollis eds, 1979) Vol. a, p. 623—629. Just some examples of $R_4$ are acyl groups such as acetyl or benzoyl, alkoxycarbonly or aryloxycarbonyl groups, silyl groups such as for example tert.-butyl dimethylsilyl, arylalkyl such as benzyl and triarylmethyl or $SO_2R_9$ where $R_9$ is as defined above.

$R_5$ is $R_1$ as defined above, or $OR_4$ or $SR_4$ where $R_4$ is defined above.

$R_4$ and $R_5$ may together form an epoxide when $R_1$ is OH and $R_4$ and $R_5$ may additionally form a cyclic derivative such as for example a carbonate ester, carbonate thioester or the corresponding ortho acid cyclic derivatives or cyclic acetal type compounds.

$R_6$ is hydroxyl, chlorine, bromine, iodine, thiol, thioether, $SO_2R_9$ where $R_9$ is as defined above; or an oxygen derivative $OR_{11}$ where $R_{11}$ is alkyl, arylalkyl such as benzyl, substituted silyl, phosphoryl diester, phosphinothioyl, or $SO_2R_9$ where $R_9$ is as defined above. $R_7$ and $R_8$ are the same or different and are hydrogen or $R_{10}$, where $R_{10}$ is an amine protecting group known to those skilled in the art and described for example in "Protective Groups in Organic Chemistry" (T. W. Greene, Wiley 1981), "Methoden der Organischen Chemie" (Houben-Weyl) Vol. XI/1 p. 1005 cont'd, or in "Comprehensive Organic Chemisty" (D. H. R. Barton and W. D. Ollis eds, 1979) Vol. 2, p. 49—52. Some examples of $R_{10}$ are acyl groups, alkoxycarbonyl or aryloxycarbonyl groups or silyl groups.

The condensation is preferably conducted in an organic solvent such as for example dimethylformamide, ethanol, acetonitrile or dichloromethane, at a temperature of between 0°C and 100°C for 1 hour to 3 days in the presence of a base (when Y is H) such as for example potassium carbonate.

After condensation, the compounds are hydrolyzed at 0—100°C for 1—24 hours with acid or base such as for example acetic acid, hydrochloric acid (1—35%) in water, sodium hydroxide (1—20%) in water, ammonium (1—25%), in water or methanol, or hydrogenated with hydrogen gas in an organic solvent such as for example ethanol or dimethylformamide over a metal catalyst for 1—24 hours at a pressure of 0.1—5 MPa.

B1. By substitution reactions on a guanine N-9 derivatized 4-carbon side-chain.

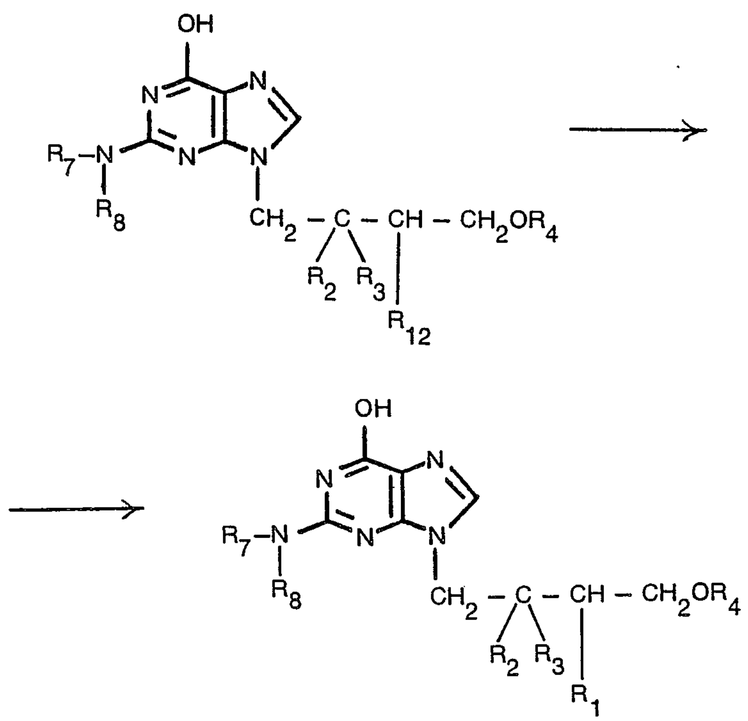

$R_2$, $R_3$, $R_4$, $R_7$ and $R_8$ are as defined above. $R_{12}$ is iodine or $OSO_2R_9$ where $R_9$ is as defined above.

The substitution reactions are performed in an organic solvent such as dimethylformamide, ethanol, acetonitrile or dichloromethane at a temperature of between 0°C and 100°C for 1—24 hours and the substituting reagent will be water, ammonia or hydrogen sulfide or their respective ions or ion pairs.

When $R_4$, $R_7$ and $R_8$ are not hydrogen then in a following reaction they are removed according to method A.

B2. By reduction of an ester group to an alcohol.

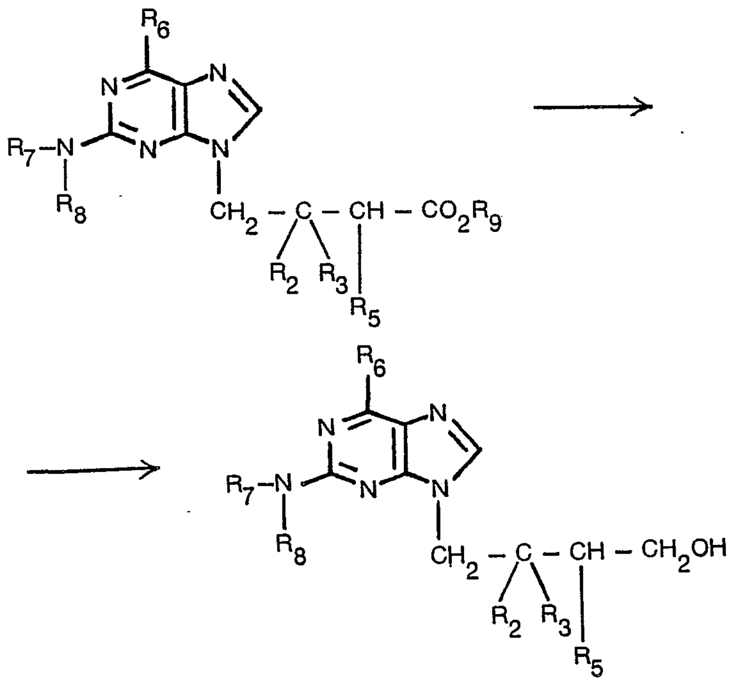

$R_2$, $R_3$, $R_5$, $R_6$, $R_7$, $R_8$ and $R_9$ are as defined above. The reduction may be performed with hydrogen gas or hydrogen generated in situ, with a metal as catalyst or with a hydride reducing agent in an organic solvent.

C1. Pyrimidine ring closure to the guanine base of a substituted imidazole derivative.

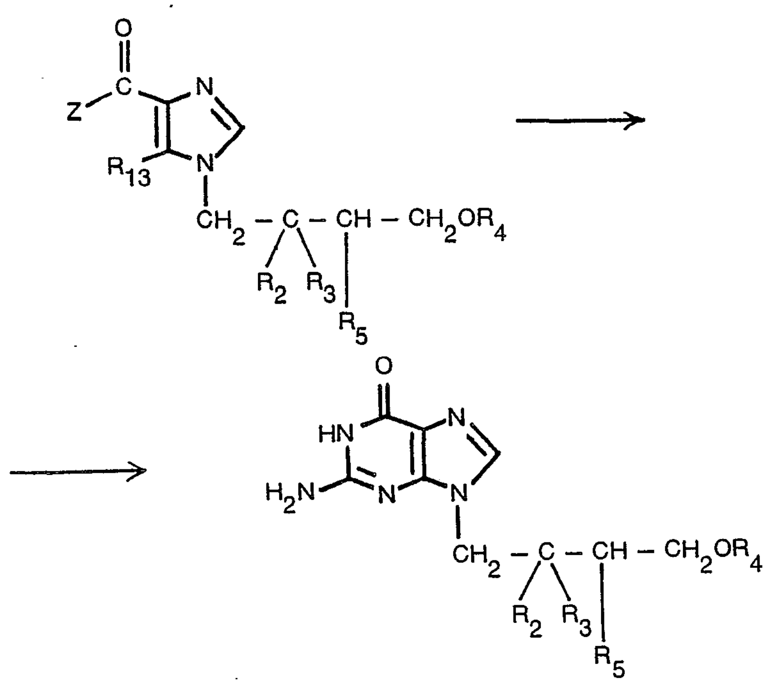

wherein $R_2$, $R_3$, $R_4$ and $R_5$ are as defined above, Z is $NH_2$ of alkoxy i.e. COZ is an amide or ester group and $R_{13}$ is $NH_2$ or guanidine. The ring closure may be performed by known methods, the principles of which are given for example in "Comprehensive Organic Chemistry" p. 505—508 (1979, Vol. 4, D. H. R. Barton and W. D. Ollis eds).

The ring closure is performed in an organic solvent at a temperature from 50° to 250°C with or without the addition of a reagent such as for example guanidine. When $R_4$ is not H and $R_5$ is not $R_1$, then the side chain protecting groups are removed in a following reaction step according to method A.

C2. Imidazole ring closure, to the guanine base, of a substituted pyrimidine derivative.

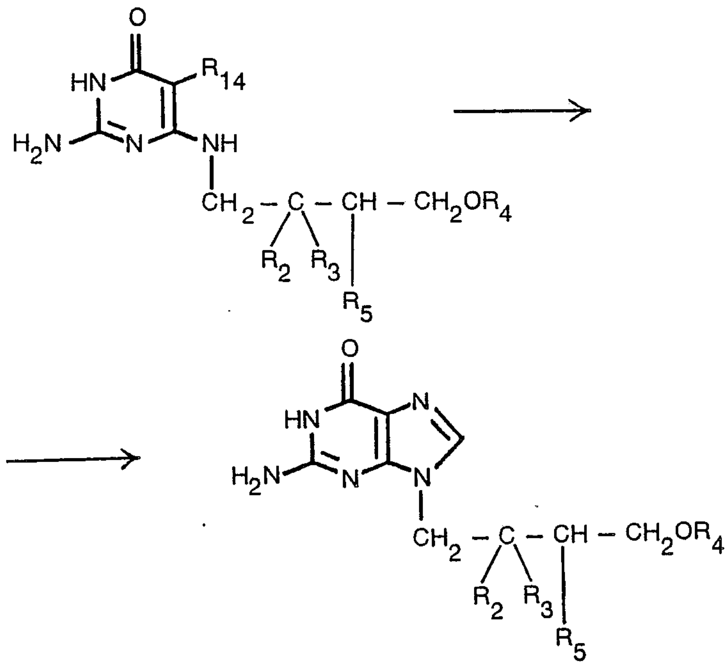

wherein $R_2$, $R_3$, $R_4$ and $R_5$ are as defined above. $R_{14}$ is nitroso, nitro, amino, or an amino derivative such as formic amide (—NH—CHO) or amino ortho ester

(e.g. 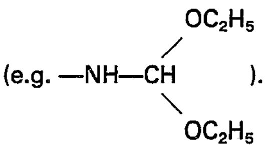 ).

The ring closure may be performed by known methods, the principles of which are given for example in "Comprehensive Organic Chemistry" p. 499—504 (1979, Vol. 4, D. H. R. Barton and W. D. Ollis eds).

The ring closure may be performed in an organic solvent such as for example formic acid, foramide, orthoformate ester at a temperature from 50 to 250°C for ½ hour to 10 hours. When $R_{14}$ is nitroso or nitro, these groups first have to be reduced to amino groups by known methods. When $R_4$ is not H and $R_5$ is not $R_1$, then the side chain protecting groups are removed in a following reaction step according to method A.

D. Substitution in the pyrimidine ring of a purine to the formation of a guanine ring.

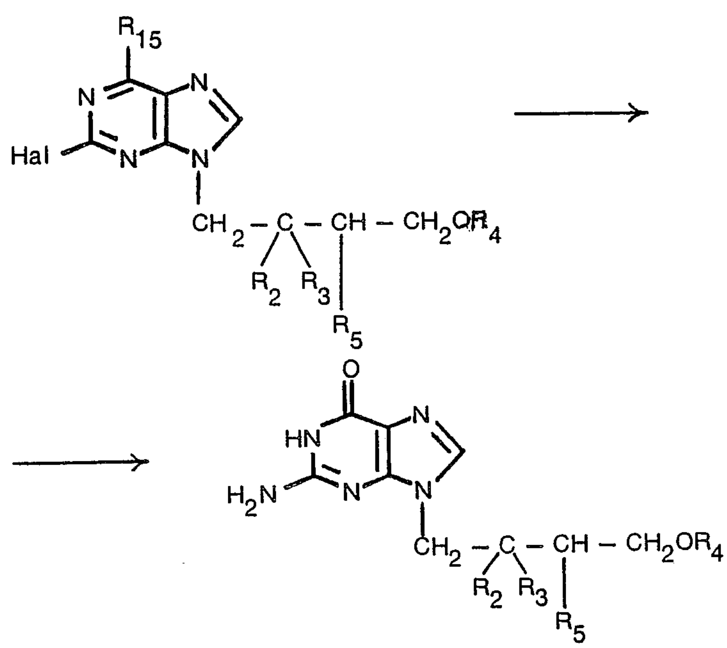

wherein $R_2$, $R_3$, $R_4$ and $R_5$ are as defined above. Hal is fluoro, chloro, bromo or iodo and $R_{15}$ is hydroxyl or amino. The halogen atoms are substituted by ammonia in an organic solvent such as methanol, from normal to higher pressure at room temperature to 100°C for 1 to 25 hours or by an azide ion followed by hydrogenation by known methods. When $R_{15}$ is amino the amino group can be substituted to a hydroxyl function by selective diazotization with nitrite in a solvent such as acetic acid at a temperature from 0°C to 50°C for 1—24 hours, or enzymatically with adenosinedeaminase in water at a pH from 6 to 9 for 1 to 48 hours. When $R_4$ is not hydrogen and $R_5$ is not $R_1$, then the side chain protecting groups are removed in a following reaction step according to method A.

The described methods A—D may be used to give enantiomeric mixtures, or in appropriate cases a single enantiomeric isomer. Additionally a single enantiomeric isomer may be obtained from the enantiomeric mixtures by methods known *per se.*

The starting material in the above methods A—D are either known compounds or can be prepared by methods known to those skilled in the art.

*Salts*

Physiologically acceptable salts of compounds of the invention are prepared by methods known in the art. The salts are novel compounds and comprise a further aspect of the invention. Metal salts can be prepared by treating a metal hydroxide with a compound of the invention. Examples of metal salts which can be prepared in this way are salts containing Li, Na and K. A less soluble metal salt can be precipitated from a solution of a more soluble salt by addition of a suitable metal compound. Acid salts can be prepared by treating a compound of the invention with an acid as HCl, HBr, $H_2SO_4$, or an organic sulphonic acid.

*Pharmaceutical compositions*

Pharmaceutical compositions of the compounds of the invention constitute a further aspect of the invention.

In clinical practice the compounds of the invention may be administered locally or systemically. They will normally be administered topically, orally, intranasally, by injection or by inhalation in the form of a pharmaceutical composition comprising the active ingredient in the form of the original compound or optionally in the form of a pharmaceutically acceptable salt thereof, in association with a pharmaceutically acceptable carrier which may be a solid, semi-solid or liquid diluent or an ingestible capsule, and such compositions comprise a further aspect of the invention. The compound may also be used without carrier material. As examples of pharmaceutical compositions may be mentioned tablets, drops, such as nasal drops, eye drops, preparations for topical application such as ointments, jellies, creams and suspensions, aerosols for inhalation, nasal spray, liposomes etc. Usually the active substance will comprise between 0.01 and 99, or between 0.1 and 99% by weight of the composition, for example between 0.5 and 20% for compositions intended for injection and between 0.1 and 50% for compositions intended for oral administration.

The compositions are preferably in dosage unit form. Further, they are preferably provided in sterilized form.

To produce pharmaceutical compositions in the form of dosage units for oral application containing a compound of the invention the active ingredient may be mixed with a solid, pulverulent carrier, for example lactose, saccharose, sorbitol, mannitol, a starch such as potato starch, corn starch, amylpectin, laminaria powder or citrus pulp powder, a cellulose derivative or gelatine and also may include lubricants such as magnesium or calcium stearate or a Carbowax® or other polyethylene glycol waxes and compressed to form tablets or cores for dragées. If dragées are required, the cores may be coated for example with concentrated sugar solutions which may contain gum arabic, talc and/or titanium dioxide, or alternatively with a film forming agent dissolved in easily volatile organic solvents or mixtures of organic solvents. Dyestuffs can be added to these coatings, for example, to distinguish between different contents of active substance. For the preparation of soft gelatine capsules consisting of gelatine and, for example, glycerol and a plasticizer, or similar closed capsules, the active substance may be admixed with a Carbowax® or a suitable oil as e.g. sesame oil, olive oil, or arachis oil. Hard gelatine capsules may contain granulates of the active substance with solid, pulverulent carriers such as lactose, saccharose, sorbitol, mannitol, starches (for example potato starch, corn starch or amylopectin), cellulose derivatives or gelatine, and may also include magnesium stearate or stearic acid as lubricants.

By using several layers of the active drug, separated by slowly dissolving coatings sustained release tablets are obtained. Another way of preparing sustained release tablets is to divide the dose of the active drug into granules with coatings of different thicknesses and compress the granules into tablets together with the carrier substance. The active substance can also be incorporated in slowly dissolving tablets made for instance of fat and wax substances or evenly distributed in a tablet of an insoluble substance such as a physiologically inert plastic substance.

Liquid compositions for oral application may be in the form of elixirs, syrups or suspensions, for example solutions containing from about 0.1% to 20% by weight of active substance, sugar and a mixture of ethanol, water, glycerol, propylene glycol and optionally aroma, saccharine and/or carboxymethylcellulose as a dispersing agent.

For parenteral application by injection compositions may comprise an aqueous solution of the active

drug or a pharmaceutically acceptable salt thereof, desirably in a concentration of 0.05—10%, and optionally also a stabilizing agent and/or buffer substances in aqueous solution. Dosage units of the solution may advantageously be enclosed in ampoules.

For topical application, especially for the treatment of herpesvirus infections on skin, genitals and in mouth and eyes the compositions are suitably in the form of a solution, ointment, gel, suspension, cream or the like. The amount of active substance may vary, for example between 0.05—20% by weight of the active substance. Such compositions for topical application may be prepared in known manner by mixing the active substance with known carrier materials such as isopropanol, glycerol, paraffin, stearyl alcohol, polyethylene glycol, etc. The pharmaceutically acceptable carrier may also include a known chemical absorption promoter. Examples of absorption promoters are e.g. dimethylacetamide (US 3,472,931), trichloroethanol or trifluoroethanol (US 3,891,757), certain alcohols and mixtures thereof (GB 1,001,949).

The dosage at which the active ingredients are administered may vary within a wide range and will depend on various factors such as for example the severity of the infection, the age of the patient, etc., and may have to be individually adjusted. As a possible range for the amount of the compounds of the invention which may be adminstered per day may be mentioned from about 0.1 mg to about 2000 mg or from about 1 mg to about 2000 mg, or preferably from 1 mg to about 2000 mg for topical administration, from 50 mg to about 2000 mg or from 100 to about 1000 mg for oral administration and from 10 mg to about 2000 mg or from 50 to about 500 mg for injection.

In severe cases it may be necessary to increase these doses 5-fold to 10-fold. In less severe cases it may be sufficient to use up to 500 or 1,000 mg.

The pharmaceutical compositions containing the active ingredients may suitably be formulated so that they provide doses within these ranges either as single dosage units or as multiple dosage units.

Thus, it has been found according to the invention that the compounds of the formula I and the physiologically acceptable salts thereof can be used to inhibit herpesvirus multiplication. The compounds of the formula I and physiologically acceptable salts thereof are useful in therapeutic and/or prophylactic treatment of virus infections.

A preferred aspect of the invention is the compounds of the formula I or physiologically acceptable salts thereof for use in the treatment of herpes virus infections.

## Working Examples

The following examples illustrate the preparation of compounds according to the invention.

### Example 1

9-(2-Oxo-4-hydroxybutyl)guanine

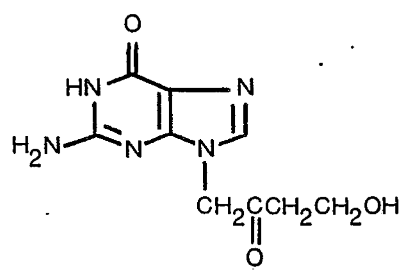

4-(2-Amino-6-chloropurin-9-yl)-3-oxobutyl o-chlorobenzoate (18 mg) was dissolved in 1M hydrochloric acid (20 ml) and refluxed for three hours. The solution was made alkaline with diluted ammoniumhydroxide and evaporated to dryness. The residue was purified by preparative HPLC on a reverse phase column (μ Bondapack $C_{18}$ methanol-water 1:3), followed by chromatography on a Biogel P-2 column eluated with water. The residue 5.4 mg was a white solid which gave a single peak on HPLC (same solvent system as above).

NMR (DMSO-$d_6$): δ 3.1 (2H, triplet, —$CH_2$—), 4.1 (2H, $NCH_2CO$) 4.3 (2H, triplet, —$CH_2$—), 6.6 (2H, singlet, $NH_2$, purine) 7.7 (1H, singlet H-8, purine).

4-(2-Amino-6-chloropurin-9-yl)-3-oxobutyl o-chlorobenzoate used as a starting material was prepared as follows:

*a) 3-Hydroxybutyl o-chlorobenzoate*

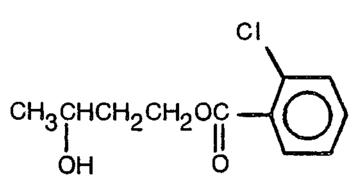

Butane-1,3-diol (1.0 g) was dissolved in pyridine (50 ml) and cooled to 0°C. Ortho-chlorobenzoylchloride (1.9 g) was added with stirring. The reaction mixture was kept at 0°C for 1 hour and then at room temperature over night. The solution was poured with stirring into ice-water (~100 ml). After stirring for 15 min the aqueous mixture was extracted with chloroform. The combined chloroform extracts were washed with ice-cold 0.25 M aqueous sulfuric acid,m saturated aqueous sodium hydrogen carbonate and water. The dried solution (sodium sulfate) was concentrated to a syrup and purified by silica gel column chromatography (toluene-ethyl acetate 1:1) to yield chromatographically pure 3-hydroxy-butyl o-chlorobenzoate (1.92 g). TLC in the same solvent $R_f$:0.56. NMR ($CDCl_3$): δ 1.24 (3H, d, $—CH_3$), 1.63—2.10 (2H, m, $—CH_2—$),

3.69—4.23 (1H, m, —CH—),
|
OH

4.29—4.66 (2H, m, $—CH_2—$), 7.13—7.52 (3H, m, aromatic protons), 7.63—7.92 (1H, m, aromatic proton).

*b) 3-Oxobutyl o-chlorobenzoate*

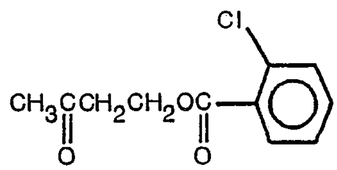

Pyridiniumchlorochromate adsorbed to aluminium oxide (33 g, 32.8 mmol) ($CrO_3 \cdot C_5H_5N \cdot HCl$ prepared according to Yu-Shia Cheng et al., Sythesis, March 1980) was added to 3-hydroxybutyl o-chlorobenzoate (1.8 g, 7.8 mmol; prepared according to a)) in n-hexane (100 ml). After stirring at room temperature over night the solid was filtered and the filtrate evaporated to dryness to give 1.21 g of 3-oxobutyl o-chlorobenzoate. TLC toluene-ethyl acetate 1:1, $R_f$ 0.74.

NMR ($CDCl_3$): δ 2.21 (3H, s $CH_3$), 2.81 (2H, triplet $—CH_2—$), 4.54 (2H, triplet $—CH_2—$), 7.0—7.45 (3H, m, aromatic protons), 7.45—7.84 (1H, m, aromatic proton).

*c) 4-Bromo-3-oxobutyl o-chlorobenzoate*

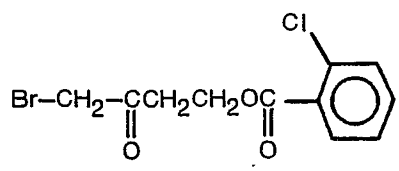

3-Oxobutyl o-chlorobenzoate (670 mg, 2.96 mmol; prepared according to b)) was dissolved in anhydrous methanol (10 ml). The solution was stirred and cooled in an icebath, and bromine (0.15 ml, 2.96 mmol) was added rapidly. The reaction mixture was kept at +5°C for 18 h. Water (7 ml) and concentrated sulfuric acid (0.6 ml) were added and the mixture was stirred at room temperature over night.

Water (10 ml) was added to the solution and the aqueous mixture was extracted with chloroform. The combined chloroform extracts were washed with saturated aqueous sodium hydrogen carbonate and water. The dried solution (sodium sulfate) was concentrated to a syrup and purified by silica gel column chromatography (toluene-ethyl acetate 16:1) to yield pure 4-bromo-3-oxobutyl o-chlorobenzoate. TLC in the same solvent, $R_f$: 0.40.

NMR ($CDCl_3$): δ 3.12 (2H, triplet $—CH_2—$),

3.87 (2H, singlet Br—$CH_2$—C),
‖
O

4.57 (2h, triplet $—CH_2—$), 7.12—7.48 (3H, m, aromatic protons), 7.48—7.81 (1H, aromatic proton).

*d) 4-(2-Amino-6-chloropurin-9-yl)-3-oxobutyl o-chlorobenzoate*

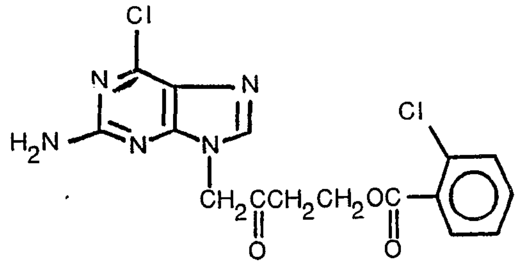

2-Amino-5-chloropurine (36.6 mg, 0.216 mmol) and anhydrous potassium carbonate (30.0 mg, 0.216 mmol) were mixed with dimethylformamide (7 ml). The mixture was cooled in an icebath and 4-bromo-3-oxobutyl o-chlorobenzoate (66 mg, 0.216 mmol; prepared according to c)) in dimethylformamide (1 ml) was added. After stirring at room temperature for 18 h, the mixture was filtered and the filtrate was evaporated to dryness. The residue was purified by preparative silica gel layer chromatography (chloroform-methanol 6:1) to yield pure 4-(2-amino-6-chloropurin-9-yl)-3-oxobutyl o-chlorobenzoate (18 mg). TLC in the same solvent, $R_f$: 040.

NMR (DMSO—$d_6$): δ 3.15 (2H, triplet —$CH_2$—), 4.26 (2H, triplet, —$CH_2$—), 5.09 (2H, singlet N—$CH_2$—CO), 6.96 (2H, S. $NH_2$ purine residue), 7.43—7.63 (3H, m, aromatic protons), 7.83—7.95 (1H, m, aromatic proton), 8.06 (1H, s, H—8, purine).

## Example 2

### 9-(2-Oxo-3,4-dihydroxybutyl)guanine

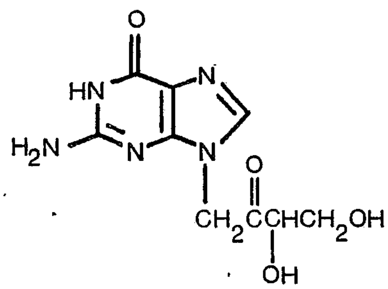

A solution of 4-(2-amino-6-chloropurin-9-yl)-3-keto-$O^1$, $O^2$-isopropylidenebutane-1,2-diol (10 mg) in 1 ml of 70% aqueous formic acid was kept at 50°C for 18 h, evaporated to dryness, neutralized with aqueous ammonia and evaporated again. Preparative HPLC ($C_{18}$ RP column, methanol-water 10:90) afforded pure 9-(2-oxo-3,4-dihydroxybutyl)guanine.

$^1$H NMR (DMSO—$d_6$): δ 3.67 (d, 2H) C<u>H</u>$_2$OH; 4.18 (t, 1H) C<u>H</u>OH; 5.10 (d, 2H) $NCH_2CO$; 6.25 (broad s, 2H) $H_2N$; 7.5 (s, 1H) $H_8$.

| | |
|---|---|
| UV (0.01 M hydrochloric acid): $\lambda_{max}$(nm) | 252, ~274 (infl.) |
| ($H_2O$, pH 7): | 250, ~270 (infl.) |
| (0.01 M sodium hydroxide): | 267, ~255 (infl.) |

4-(2-Amino-6-chloropurin-9-yl)-3-keto-$O^1$, $O^2$-iospropylidenebutane-1,2-diol used as a starting material was prepared as follows:

*a) 4-Chloroacetyl-2,2-dimethyldioxolane*

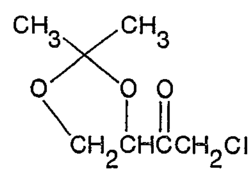

A solution of glyceroyl chloride acetonide [0.22 g, 1.32 mmol; T. Reichstein, A. Pedolin and A. Grüssner, Helv. Chim. Acta 18, 598 (1935)] in 2 ml of diethyl ether was added with stirring to a solution of diazomethane (2.7 mmol) in 10 ml of diethyl ether. After 1 h a solution of hydrogen chloride in diethyl ether

(1 ml, 3.5 M) was added with stirring at room temperature, the solution was neutralized and dried with solid potassium carbonate and evaporated in vacuum to a small volume. The light yellow oil (146 mg, 62%) was used directly in the following step.

$^1H$ NMR ($CDCl_3$): δ 1.40 and 1.52 (2s, 2×3H) $C(CH_3)_2$; 3.8—4.8 (m, 3H) $CHCH_2$; 4.48 (s, 2H) $CH_2Cl$.

*b) 4-(2-Amino-6-chloropurin-9-yl)-3-keto-*$O^1$, $O^2$*-iopropylidenebutane-1,2-diol*

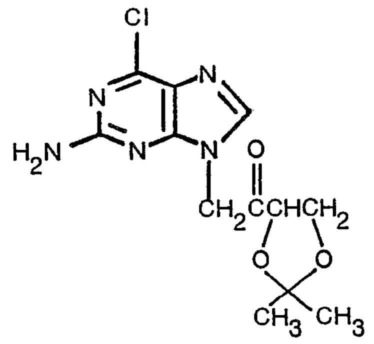

A mixture of 2-amino-6-chloropurine (277 mg, 1.635 mmol), finely ground, anhydrous potassium carbonate (226 mg, 1.635 mmol) and 20 ml of dry dimethylformamide was warmed to 100°C for 5 min, then cooled, finally in ice-water. A solution of 4-chloroacetyl-2,2-dimethyldioxolane (146 mg, 0.817 mmol; prepared according to a)) in 5 ml of DMF was added dropwise with stirring at 0°C and the mixture was stirred at ambient temperature for 18 h. The solvent was evaporated in vacuum. Chromatography (150 g of silica gel, $CHCl_3$—MeOH 9:1 by volume) gave 31 mg (14%) of 4-(2-amino-6-chloropurin-9-yl)-3-keto-$O^1$, $O^2$-isopropylidenebutane-1,2-diol.

$^1H$ NMR ($CDCl_3$): 1.45 and 1.62 (2s, 2×3H) $C(CH_3)_2$; 4.2—4.4 and 4.67 (ABX system, $J_{vic}$ 3Hz and 7Hz) $CH_2CH$; 5.15 (broad s, 2H) $H_2N$; 5.18 (s, 2H) $NCH_2$; 7.77 (s, 1H) $H_8$.

The following examples illustrate the preparation of pharmaceutical compositions of the invention. The wording "active substance" denotes a compound according to the present invention of a salt thereof.

**Tablets**

Each tablet contains:

| | |
|---|---|
| Active substance | 20.0 mg |
| Maize starch | 25.0 mg |
| Lactose | 190.0 mg |
| Gelatin | 1.5 mg |
| Talc | 12.0 mg |
| Magnesium stearate | 1.5 mg |
| | 250.0 mg |

**Suppositories**

Each suppository contains:

| | |
|---|---|
| Active substance | 20.0 mg |
| Ascorbyl palmitate | 1.0 mg |
| Suppository base (Imhausen H or Witepsol®) ad | 2000.0 mg |

Syrup

| | |
|---|---|
| Active substance | 0.200 g |
| Liquid glucose | 30.0 g |
| Sucrose | 50.0 g |
| Ascorbic acid | 0.1 g |
| Sodium pyrosulfite | 0.01 g |
| Disodium edetate | 0.01 g |
| Orange essence | 0.025 g |
| Certified colour | 0.015 g |
| Purified water | ad 100.0 g |

Injection solution

| | |
|---|---|
| Active substance | 3.000 mg |
| Sodium pyrosulfite | 0.500 mg |
| Disodium edetate | 0.100 mg |
| Sodium chloride | 8.500 mg |
| Sterile water for injection | ad 1.00 ml |

Sublingual tablets

| | |
|---|---|
| Active substance | 5.0 mg |
| Lactose | 85.0 mg |
| Talc | 5.0 mg |
| Agar | 5.0 mg |
| | 100.0 mg |

Jelly

| | |
|---|---|
| Active substance | 1.0 g |
| Methocel® | 4.0 g |
| Methyl paraoxybenzoate | 0.12 g |
| Propyl paraoxybenzoate | 0.05 g |
| Sodium hydroxide and hydrochloric acid to pH 6.7 | |
| Distilled water | ad 100.0 ml |

Ointment I

| | |
|---|---|
| Active substance | 1.0 g |
| Cetyltrimethylammoniumbromide | 0.6 g |
| Stearyl alcohol | 2.25 g |
| Cetanol | 6.75 g |
| Liquid paraffine | 17.0 g |
| Glycerol | 12.0 g |
| Hydrochloric acid to pH 6.5 | |
| Distilled water | ad 100.0 g |

Ointment II

| | |
|---|---|
| Active substance | 3.0 g |
| Polyethylene glycol 1500 | 50.0 g |
| Polyethylene glycol 4000 | 15.0 g |
| Propylene glycol | ad 100.0 g |

Ointment III

| | |
|---|---|
| Active substance | 3.0 g |
| Sorbitan monoleate | 5.0 g |
| Petrolatum | ad 100.0 g |

Ointment IV

| | |
|---|---|
| Active substance | 5.0 g |
| Adeps Tanae | 20.0 g |
| Tween® 60 | 4.0 g |
| Span® 40 | 2.0 g |
| Paraffin, liquid | 4.0 g |
| Propylene glycol | 5.0 g |
| Hydrochloric acid to pH 6.5—8 | |
| Sterile water | ad 100.0 g |

Ointment V

| | |
|---|---|
| Active substance | 5.0 g |
| Adeps Tanae | 20.0 g |
| Tween® 60 | 4.0 g |
| Span® 40 | 2.0 g |
| Paraffin, liquid | 4.0 g |
| Propylene glycol | 5.0 g |
| Boric acid | 2.0 g |
| Sodium hydroxide to pH 6.5—8 | |
| Sterile water | ad 100.0 g |

Eye drops I

| | |
|---|---|
| Active substance | 0.1 g |
| Disodium edetate | 0.10 g |
| Sodium chloride for isotonia q.s. | |
| Hydrochloric acid to pH 6.5—8 | |
| Methocel® 65 HG 4000 | 0.65 g |
| Sterile water | ad 100.0 ml |

Eye drops II

| | |
|---|---|
| Active substance | 0.3 g |
| Disodium edetate | 0.10 g |
| Sodium chloride for isotonia q.s. | |
| Hydrochloric acid to pH 6.5—8 | |
| Methocel® 65 HG 4000 | 0.65 g |
| Sterile water | ad 100.0 ml |

Eye drops III

| | |
|---|---|
| Active substance | 0.2 g |
| Disodium edetate | 0.1 g |
| Sodium chloride for isotonia q.s. | |
| Boric acid | 0.1 g |
| Methocel® 65 HG 4000 | 0.65 g |
| Sterile water | ad 100.0 ml |

Eye ointment I

| | |
|---|---|
| Active substance | 3.0 g |
| Paraffin oil | 19.0 g |
| Petrolatum | 78.0 g |

Cream

| | |
|---|---|
| Active substance | 3.0 g |
| Arlaton® | 4.0 g |
| Cetanol | 2.0 g |
| Stearic acid | 2.0 g |
| Paraffin oil | 2.0 g |
| Propylene glycol | 2.0 g |
| Glycerol | 1.5 g |
| Methyl p-hydroxybenzoate | 0.06 g |
| Propyl p-hydroxybenzoate | 0.03 g |
| Sodium hydroxide | 0.002 g |
| Hydrochloric acid 2M to pH 8.0 (water phase) | |
| Distilled water | to 100.0 g |

Jelly

| | |
|---|---|
| Active substance | 3.0 g |
| Methocel® | 2.45 g |
| Glycerol | 10.0 g |
| Tween® | 0.10 g |
| Methyl p-hydroxybenzoate | 0.06 g |
| Propyl p-hydroxybenzoate | 0.03 g |
| Sodium hydroxide | 0.002 g |
| Hydrochloric acid 2M to pH 8.0 | |
| Distilled water | to 100.0 g |

Tablets

Each tablet contains:

| | |
|---|---|
| Active substance | 100.0 mg |
| Starch | 60.0 mg |
| Lactose | 190.0 mg |
| Polyvinylpyrrolidone | 5.0 mg |
| Magnesium stearate | 5.0 mg |
| | 360.0 mg |

Biological tests

The inhibiting effect of compounds of the invention on herpesvirus was tested using the method described below. The cellular toxicity of the compounds on host cell functions was also tested.

The following compounds were tested:
9-(2-oxo-4-hydroxybutyl)guanine VIS 590
9-(2-oxo-3,4-dihydroxybutyl)guanine VSA 687

I. Inhibition of virus multiplication in cell cultures

The inhibition of herpesvirus by VIS 590 and VSA 687 has been measured as plaque reduction according to the following procedure.

The plaque reduction assay for herpes simplex type 1 was performed on Vero (Green Monkey Kidney) cells as described by Ejercito et al., J. Gen. Virol. 2 (1968) 357. Monolayers on 5 cm petri dishes were used and after virus adsorption the compounds to be tested were added to the medium. The results are shown in table 1.

Table 1. Inhibition of herpes simplex type 1 plaque on Vero cell mono-layers

| Conc. µ | Test compound | Inhibition % |
|---|---|---|
| 10 | VIS 590 | 35 |
| 100 | VIS 590 | >90 |
| 35 | VSA 687 | >90 |

II. Cellular toxicity

VIS 590 was tested for cellular toxicity as shown in Table 2. The method used has been described by Stenberg (Biochemical Pharmacology, Vol. 30 (1980), 1005—1008). It is seen that VIS 590 did not affect cell growth at 100 µM. However, at this concentration, herpes virus multiplication was inhibited to more than 90% (see Table 1).

Table 2. Cellular toxicity of VIS 590 expressed as percent reduction in cell growth after 48 h of incubation.

| Conc. µM | Test compound | Percent reduction in growth of Vero cells, determined as cell number |
|---|---|---|
| 100 | VIS 590 | 0 |

**Claims**

1. A compound of the formula

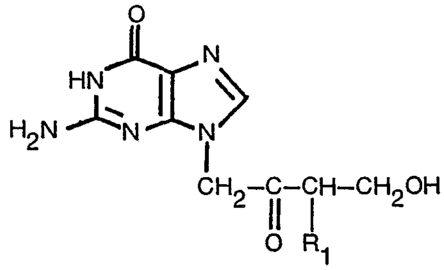

I

wherein $R_1$ is hydrogen, hydroxy or mercapto; and physiologically acceptable salts or optical isomers thereof.

2. A compound according to claim 1, wherein $R_1$ is hydrogen, 9-(2-oxo-4-hydroxybutyl)guanine; and physiologically acceptable salts thereof.

3. A compound according to claim 1, wherein $R_1$ is hydroxy, 9-(2-oxo-3,4-dihydroxybutyl)guanine; and physiologically acceptable salts or optical isomers thereof.

4. A compound according to claim 1, wherein $R_1$ is mercapto, 9-(2-oxo-3-mercapto-4-hydroxybutyl)guanine; and physiologically acceptable salts or optical isomers thereof.

5. A pharmaceutical composition comprising as an active ingredient a compound of the formula

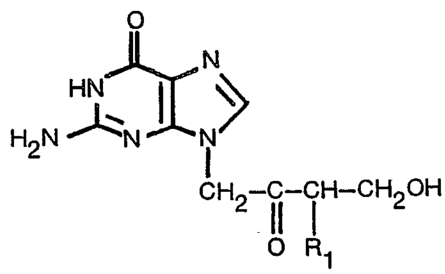

I

wherein $R_1$ is hydrogen, hydroxy or mercapto; or a physiologically acceptable salt or an optical isomer thereof; in conjunction with a pharmaceutically acceptable carrier.

6. A pharmaceutical composition according to claim 5 designed for systemic administration.

7. A pharmaceutical composition according to claim 5 designed for topical administration.

8. A compound according to any of claims 1—4 for use in the treatment of virus infections.

9. A compound according to claim 8 for use in the treatment of herpes virus infections.

10. A compound according to any of claims 1—4 for use in the treatment of neoplastic diseases.

11. A method for the preparation of a compound of the formula

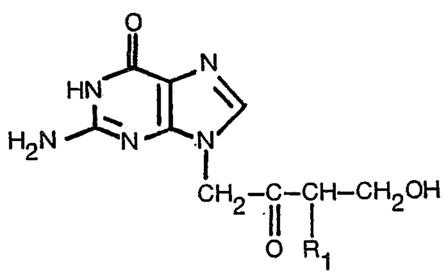

I

wherein $R_1$ is hydrogen, hydroxy or mercapto; or a physiologically acceptable salt or an opticl isomer thereof comprising:

A. Condensing an acyclic side chain of the formula

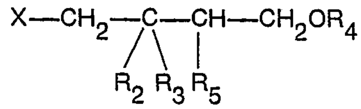

to the N-9 position of a guanine derivative of the formula

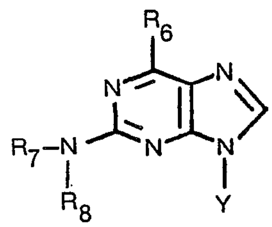

followed by removal of possible protecting groups, in which formulas X is a chlorine, bromine, iodine or a group $OSO_2R_9$ where $R_9$ is alkyl containing 1—8 carbon atoms, fluorinated alkyl containing 1—8 carbon atoms, aryl or alkylaryl, Y is hydrogen or a quaternary ammonium ion; $R_2$ and $R_3$ are together double-bonded oxygen, or alkoxy groups forming open or cyclic ketals; $R_4$ is hydrogen or a hydroxyl protecting group; $R_5$ is $R_1$ as defined above; $R_4$ and $R_5$ may optionally together form an epoxide when $R_1$ is OH, or $R_4$ and $R_5$ may optionally form a cyclic derivative such as a carbonate ester, carbonate thioester or the corresponding ortho acid cyclic derivatives or cyclic acetal type compounds; $R_6$ is hydroxyl, chlorine, bromine, iodine, thiol, thioether, $SO_2R_9$ is as defined above; or an oxygen derivative $OR_{11}$ where $R_{11}$ is alkyl, arylalkyl such as benzyl, substituted silyl, phosphoryl diester, phospinothioyl, or $SO_2R_9$ where $R_9$ is as defined above; $R_7$ and $R_8$ are the same or different and are hydrogen or $R_{10}$, where $R_{10}$ is as defined above; or

B1. Substitution in the guanine N-9 derivatized 4-carbon side-chain of a compound of the formula

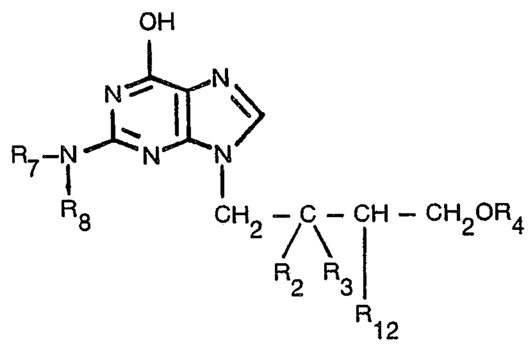

to form a compound of the formula

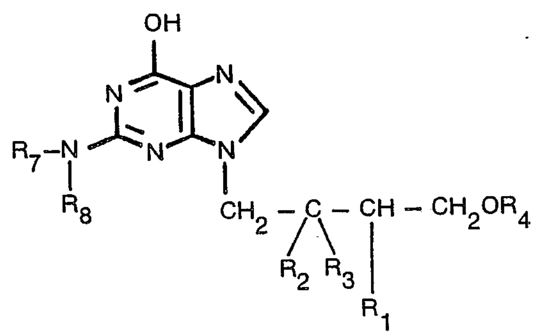

followed by removal of possible protecting groups, in which formula $R_2$, $R_3$, $R_4$, $R_7$ and $R_8$ are as defined above, and $R_{12}$ is iodine or $OSO_2R_9$ where $R_9$ is as defined above; or

B2. Reducing the ester group to an alcohol in a compound of the formula

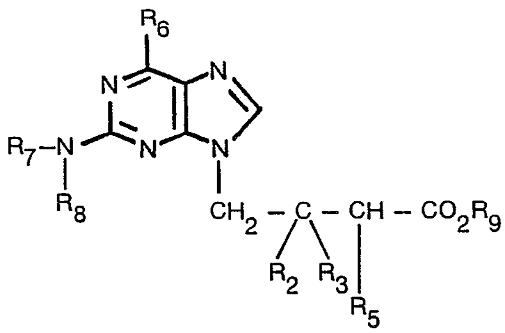

followed by removal of possible protecting groups, in which formula $R_2$, $R_3$, $R_5$, $R_6$, $R_7$ $R_8$ and $R_9$ are as defined above; or

C1. Ring closure of a compound of the formula

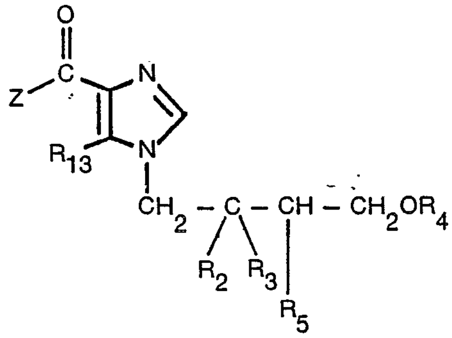

followed by removal of possible protecting groups, in which formula $R_2$, $R_3$, $R_4$ and $R_5$ are as defined above, Z is $NH_2$ or an alkoxy group; and $R_{13}$ is $NH_2$ or guanidine; or

C2. Ring closure or a compound of the formula

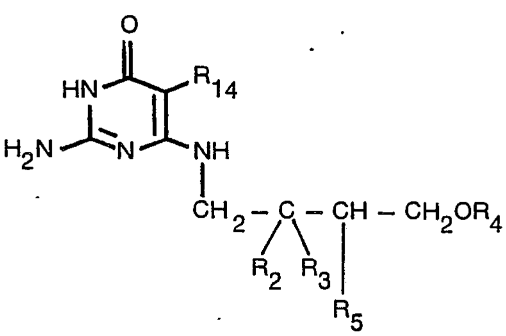

followed by removal of possible protecting groups, in which formula $R_2$, $R_3$, $R_4$ and $R_5$ are as defined above, and $R_{14}$ is nitroso, nitro, amino, formic amide or amino ortho ester; or

D. Substitution in the pyrimidine ring of a compound of the formula

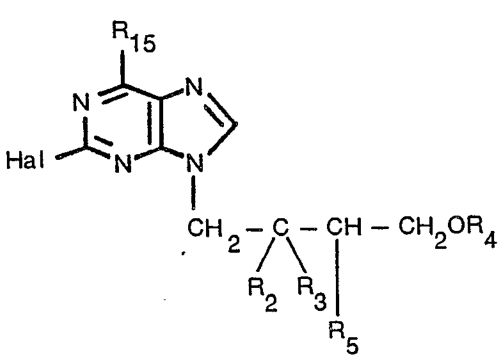

to form a guanine ring

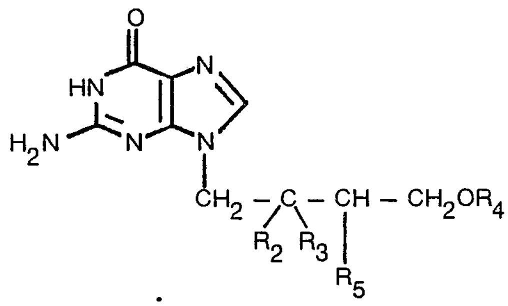

followed by removal of possible protecting groups, in which formula $R_2$, $R_3$, $R_4$ and $R_5$ are as defined above, Hal is fluoro, chloro, bromo, or iodine, and $R_{15}$ is hydroxyl or amino, whereby when $R_{15}$ is amino the amino group is converted to a hydroxyl function; to form a compound of the formula I, whereupon optionally a base obtained is converted to a pharmaceutically acceptable salt or a salt obtained is converted to the base or to a different, pharmaceutically acceptable salt, and optionally an isomeric mixture obtained is separated into a pure enantiomeric isomer.

12. A method according to claim 11 for the preparation of a compound of any of claims 2—4.

13. A compound according to any of claims 1—4 for use as a therapeutic agent.

14. A compound according to any of claims 1—4 for use as a prophlyactic agent in the medical field.

**Patentansprüche**

1. Verbindung der Formel

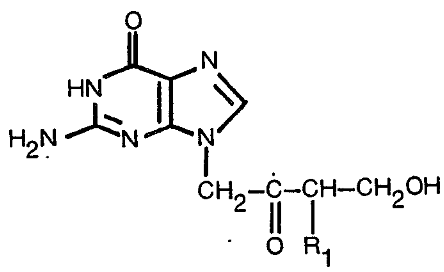

I

worin $R_1$ Wasserstoff, Hydroxy oder Mercapto bedeutet, und physiologisch akzeptable Salze oder optische Isomere derselben.

2. Verbindung nach Anspruch 1, worin $R_1$ Wasserstoff bedeutet, d.h. 9-(2-Oxo-4-hydroxybutyl)-guanin, und physiologisch akzeptable Salze davon.

3. Verbindung nach Anspruch 1, worin $R_1$ Hydroxy bedeutet, d.h. 9-(2-Oxo-3,4-dihydroxybutyl)-guanin, und physiologisch akzeptable Salze oder optische Isomere davon.

4. Verbindung nach Anspruch 1, worin $R_1$ Mercapto bedeutet, d.h. 9-(2-Oxo-3-mercapto-4-hydroxybutyl)-guanin, und physiologisch akzeptable Salze oder optische Isomere davon.

5. Pharmazeutische Zusammensetzung, die als aktives Ingrediens eine Verbindung der Formel

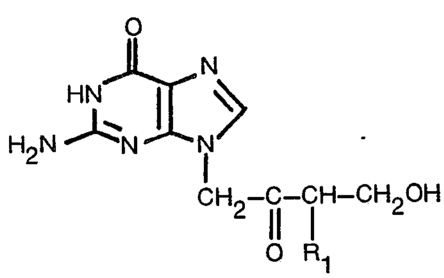

I

worin $R_1$ Wasserstoff, Hydroxy oder Mercapto bedeutet, oder ein physiologisch akzeptables Salz oder ein optisches Isomeres davon in Verbindung mit einem pharmazeutisch akzeptablen Träger enthält.

6. Pharmazeutische Zusammensetzung nach Anspruch 5 für systemische Verabreichung.

7. Pharmazeutische Zusammensetzung nach Anspruch 5 für topische Verabreichung.

8. Verbindung nach einem der Ansprüche 1 bis 4 zur Verwendung bei der Behandlung von Virusinfektionen.

9. Verbindung nach Anspruch 8 zur Verwendung bei der Behandlung von Herpesvirusinfektionen.

10. Verbindung nach einem der Ansprüche 1 bis 4 zur Verwendung bei der Behandlung neoplastischer Erkrankungen.

11. Verfahren zur Herstellung einer Verbindung der Formel

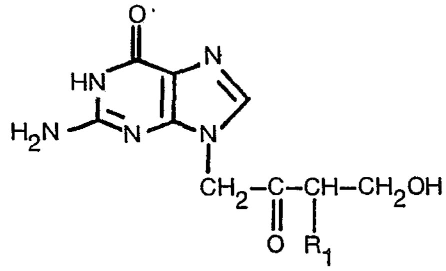

I

worin $R_1$ Wasserstoff, Hydroxy oder Mercapto bedeutet, oder eines physiologisch akzeptablen Salzes oder optischen Isomeren davon, gekennzeichnet durch:

A. Kondensieren einer azyklischen Seitenkette der Formel

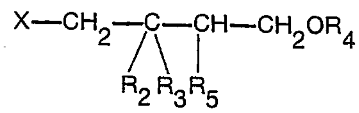

zur N-9-Position eines Guaninderivates der Formel

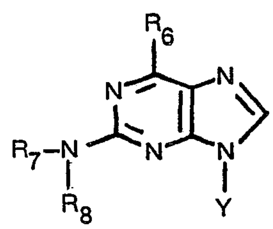

mit anschließendem Entfernen möglicher Schutzgruppen, in welchen Formeln X Chlor, Brom, Jod oder einer Gruppe $OSO_2R_9$, worin $R_9$ Alkyl mit 1 bis 8 Kohlenstoffatomen, fluoriniertes Alkyl mit 1 bis 8 Kohlenstoffatomen, Aryl oder Alkylaryl ist, bedeutet, Y für Wasserstoff oder ein quaternäres Ammoniumion steht, $R_2$ und $R_3$ zusammen doppeltgebunden Sauerstoff oder offene oder zyklische Ketale bildende Alkoxygruppen darstellen, $R_4$ Wasserstoff oder eine Hydroxyl-Schutzgruppe bedeutet, $R_5$ $R_1$ ist, das obige Bedeutung hat, $R_4$ und $R_5$ gegebenenfalls zusammen ein Epoxid bilden können, wenn $R_1$ für OH steht, oder $R_4$ und $R_5$ gegebenenfalls ein zyklisches Derivat, wie Carbonatester, Carbonatthioester oder die entsprechenden zyklischen Orthosäurederivate oder zyklischen Verbindungen des Acetaltyps bilden können, $R_6$ für Hydroxyl, Chlor, Brom, Jod, Thiol, Thioäther, $SO_2R_9$, worin $R_9$ obige bedeutung hat, oder ein Sauerstoffderivat $OR_{11}$, worin $R_{11}$ Alkyl, Arylalkyl, wie Benzyl, substituiertes Silyl, Phosphoryldiester, Phosphinothioyl oder $SO_2R_9$, worin $R_9$ obige Bedeutung hat, darstellt, steht, $R_7$ und $R_8$ gleich oder verschieden sind und für Wasserstoff oder $R_{10}$, worin $R_{10}$ obige bedeutung hat, stehen; oder

B1. Substituieren in der Guanin N-9 derivatisierten 4-Kohlenstoff-Seitenkette einer Verbindung der Formel

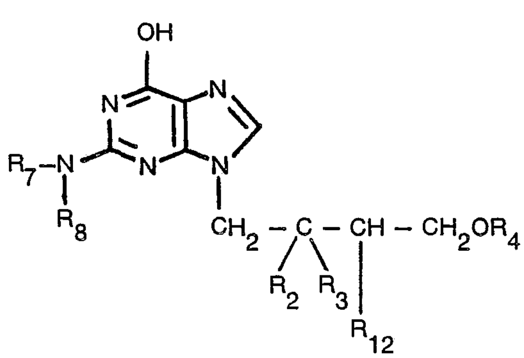

zur Bildung einer Verbindung der Formel

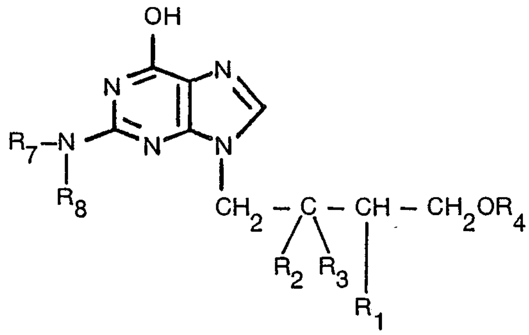

mit anschließendem Entfernen möglicher Schutzgruppen, in welcher Formel $R_2$, $R_3$, $R_4$, $R_7$ und $R_8$ obige bedeutung haben, und $R_{12}$ Jod oder $OSO_2R_9$, worin $R_9$ obige Bedeutung hat, ist; oder

B2. Reduzieren der Estergruppe zu einem Alkohol in einer Verbindung der Formel

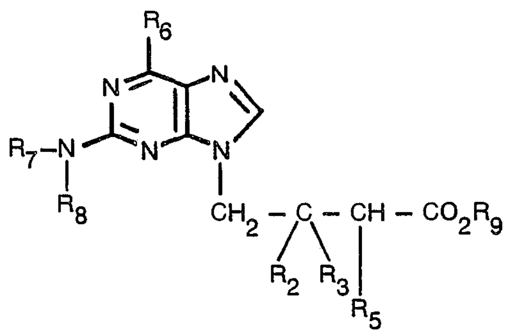

mit anschließendem Entfernen möglicher Schutzgruppen, in welcher Formel $R_2$, $R_3$, $R_5$, $R_6$. $R_7$, $R_8$ und $R_9$ obige Bedeutung haben; oder

C1. Ringschluß einer Verbindung der Formel

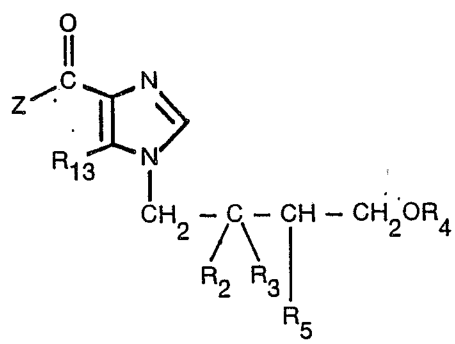

mit anschließendem Entfernen möglicher Schutzgruppen, in welcher Formel $R_2$, $R_3$, $R_4$ und $R_5$ obige Bedeutung haben, Z für $NH_2$ oder eine Alkoxygruppe steht und $R_{13}$ $NH_2$ oder Guanidin bedeutet; oder

C2. Ringschluß einer Verbindung der Formel

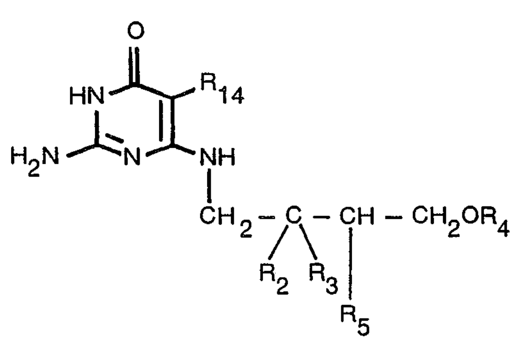

mit anschließendem Entfernen möglicher Schutzgruppen, in welcher Formel $R_2$, $R_3$, $R_4$ und $R_5$ obige Bedeutung haben und $R_{14}$ Nitroso, Nitro, Amino, Formamid, oder Amino-orthoester darstellt; oder

D. Substituieren einer Verbindung der Formel

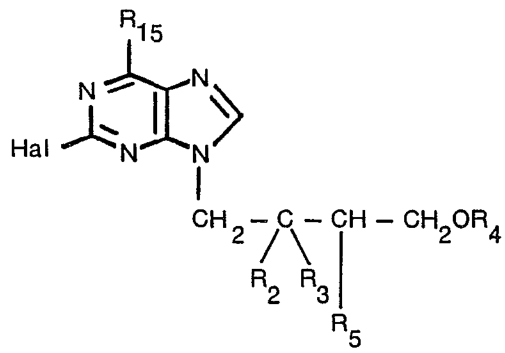

im Pyrimidinring zur Bildung eines Guaninrings

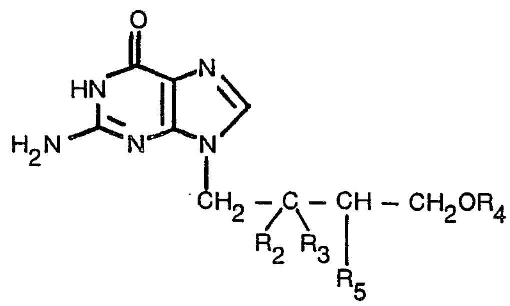

mit anschließendem Entfernen möglicher Schutzgruppen, in welcher Formel $R_2$, $R_3$, $R_4$ und $R_5$ obige Bedeutung haben, Hal für Fluor, Chlor, Brom oder Jod steht und $R_{15}$ Hydroxyl oder Amino bedeutet, wobei für den Fall, daß $R_{15}$ Amino ist, die Aminogruppe in eine Hydroxylfunktion umgewandelt wird; zu einer Verbindung der Formel I, worauf gegebenenfalls eine erhaltene Base in ein pharmazeutisch akzeptables Salz bzw. ein erhaltenes Salz in die Base oder in ein anderes pharmazeutisch akzeptables Salz übergeführt und gegebenenfalls eine erhaltene Isomerenmischung in ein reines enantiomeres Isomeres aufgetrennt wird.

12. Verfahren nach Anspruch 11 zur Herstellung einer Verbindung nach einem der Ansprüche 2 bis 4.

13. Verbindung nach einem der Ansprüche 1 bis 4 zur Verwendung als therapeutisches Mittel.

14. Verbindung nach einem der Ansprüche 1 bis 4 zur Verwendung als prophylaktischen Mittel in der Medizin.

**Revendications**

1. Composé de formule

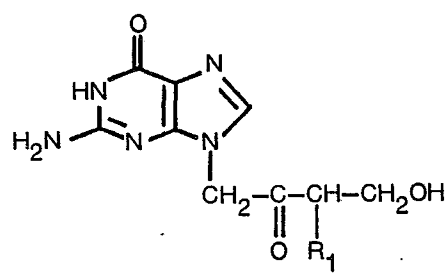

I

dans laquelle $R_1$ est l'hydrogène, le radical hydroxyle ou mercapto, et ses sels ou isomères optiques physiologiquement acceptables.

2. Composé selon la revendication 1, dans lequel $R_1$ est l'hydrogène, la 9-(2-oxo-4-hydroxybutyl)guanine, et ses sels physiquement acceptables.

3. Composé selon la revendication 1, dans lequel $R_1$ est le radical hydroxyle, la 9-(2-oxo-3,4-dihydroxybutyl)guanine, et ses sels ou isomères optiques physiologiquement acceptables.

4. Composé selon la revendication 1, dans lequel $R_1$ est le radical mercapto, la 9-(2-oxo-3-mercapto-4-hydroxybutyl)-guanine, et ses sels ou isomères optiques physiologiquement acceptables.

5. Composition pharmaceutique comprenant en tant que principe actif un composé de formule

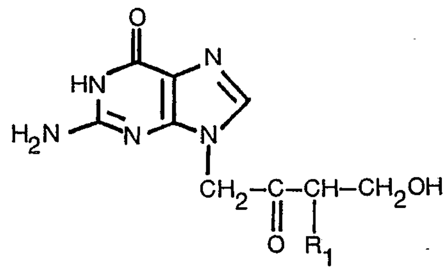

I

dans laquelle $R_1$ est l'hydrogène, le radical hydroxyle ou mercapto, ou l'un de ses sels ou isomères optiques physiologiquement acceptables, en liaison avec un excipient pharmaceutiquement acceptable.

6. Composition pharmaceutique selon la revendication 5, destinée à une administration générale.

7. Composition pharmaceutique selon la revendication 5, destinée à une administration locale.

8. Composé selon l'une quelconque des revendications 1—4, pour une utilisation dans le traitement des infections virales.

9. Composé selon la revendication 8, pour une utilisation dans le traitement des infections par herpèsvirus.

10. Composé sslon l'une quelconque des revendications 1—4, pour utilisation dans le traitement des maladies néoplastiques.

11. Procédé pour la préparation d'un composé de formule

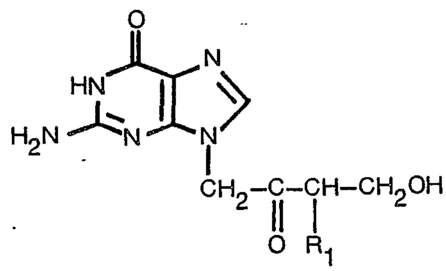

I

dans laquelle $R_1$ est l'hydrogène, le radical hydroxyle ou mercapto, ou d'un de ses sels ou isomères optiques physiologiquement acceptables, comprenant:

A. La condensation d'une chaîne latérale acyclique de formule

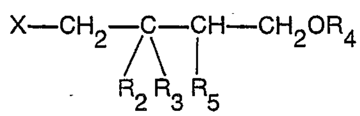

sur la position N—9 d'un dérivé de guanine de formule

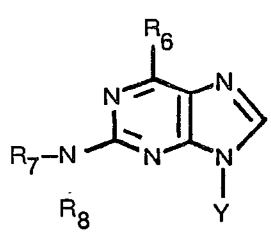

suivie de l'élimination des éventuels groupes protecteurs, formules dans lesquelles X est un atome de chlore, de brome, d'iode ou un groupe $OSO_2R_9$ dans lequel $R_9$ est un radical alkyle ayant de 1 à 8 atomes de carbone, alkyle fluoré ayant 1 à 8 atomes de carbone, aryle ou alkylaryle; Y est l'hydrogène ou un ion ammonium quaternaire; $R_2$ et $R_3$ forment ensemble un oxygène à double liaison, ou des groupes alcoxy formant des cétals ouverts ou cycliques; $R_4$ est l'hydrogène ou un groupe protecteur d'hydroxyle; $R_5$ est le radical $R_1$ tel que défini ci-dessus; $R_4$ et $R_5$ peuvent facultativement former ensemble un radical époxyde quand $R_1$ est OH, ou bien $R_4$ et $R_5$ peuvent facultativement former un dérivé cyclique tel qu'un ester carbonique, un thioester carbonique ou les dérivés cycliques des orthoacides ou les composés cycliques du type acétal correspondants; $R_6$ est le radical hydroxyle, le chlore, le brome, l'iode, le groupe thiol, un

groupe thioéther, $SO_2R_9$ où $R_9$ est comme il a été défini ci-dessus; ou bien un dérivé oxygéné $OR_{11}$ dans lequel $R_{11}$ est un radical alkyle, arylalkyle, tel que le radical benzyle, silyle substitué, diester phosphorylique, phosphinothioyle, ou $SO_2R_9$ où $R_9$ est comme il a été défini ci-dessus; $R_7$ et $R_8$ sont identiques ou différents et sont l'hydrogène ou $R_{10}$, où $R_{10}$ est comme il a été defini ci-dessus; ou bien.

B1. Substitution, dans le chaîne latérale à 4 atomes de carbone fixée sur l'atome N-9 de la guanine, d'un composé de formule

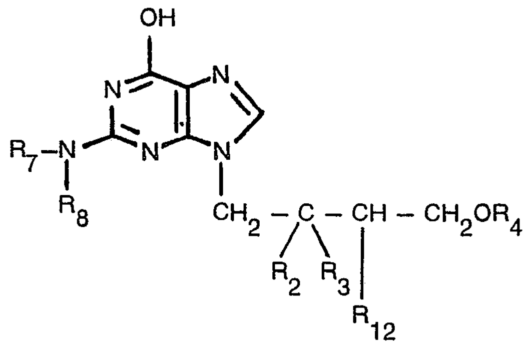

pour obtenir un composé de formule

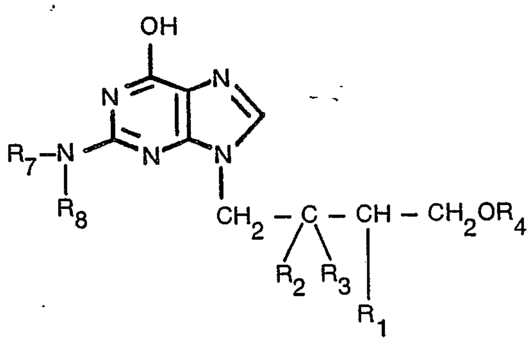

suivie de l'élimination des éventuels groupes protecteurs, formule dans laquelle $R_2$, $R_3$, $R_4$, $R_7$ et $R_8$ sont comme ils ont été définis ci-dessus, et $R_{12}$ est l'iode ou $OSO_2R_9$ où $R_9$ est comme il a été défini ci-dessus; ou bien.

B2. Réduction du groupe ester en un alcool dans un composé de formule

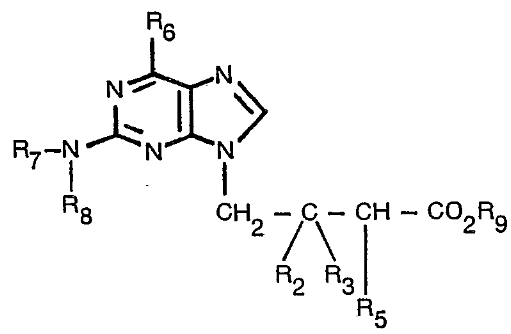

suivie de l'élimination des éventuels groupes protecteurs, formule dans laquelle $R_2$, $R_3$, $R_5$, $R_6$, $R_7$, $R_8$ et $R_9$ sont comme ils ont été définis ci-dessus; ou bien.

C1. Cyclisation d'un composé de formule

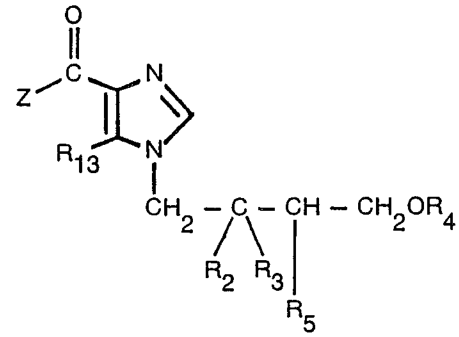

suivie de l'élimination des éventuels groupes protecteurs, formule dans laquelle $R_2$, $R_3$, $R_4$ et $R_5$ sont comme ils ont été définis ci-dessus, Z est $NH_2$ ou un groupe alcoxy, et $R_{13}$ est $NH_2$ ou la guanidine; ou bien.

C2. Cyclisation d'un composé de formule

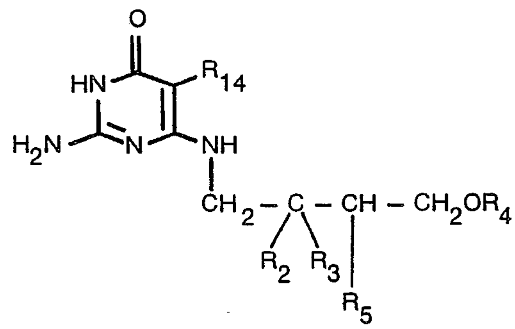

suivie de l'élimination des éventuels groupes protecteurs, formule dans laquelle $R_2$, $R_3$, $R_4$ et $R_5$ sont comme ils ont été définis ci-dessus, et $R_{14}$ est un radical nitroso, nitro, amino, amide formique ou amino-orthoester; ou bien.

D. Substitution, dans le noyau pyrimidine, d'un composé de formule

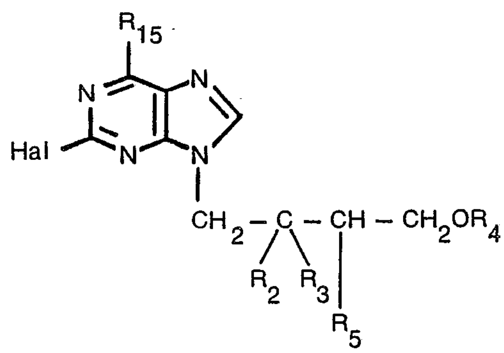

pour former un noyau guanine

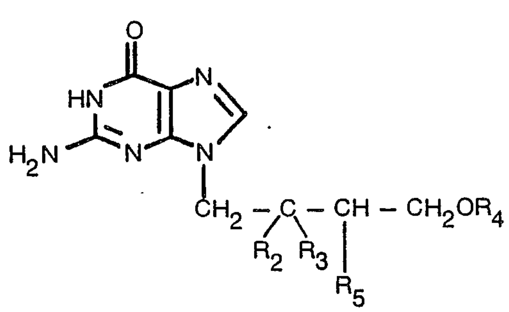

suivie de l'élimination des éventuels groupes protecteurs, formule dans laquelle $R_2$, $R_3$, $R_4$ et $R_5$ sont comme ils ont été définis ci-dessus, Hal est le radical fluoro, chloro, bromo ou iodo, et $R_{15}$ est le groupe hydroxyle ou amino, et où, quand $R_{15}$ est le groupe amino, le groupe amino est converti en une fonction hydroxyle;

pour former un composé de formule I, ce après quoi, éventuellement, une base obtenue est convertie en un sel pharmaceutiquement acceptable, ou bien un sel obtenu est converti en la base ou en autre sel pharmaceutiquement acceptable, et, facultativement, un mélange isomère obtenu est séparé pour donner un isomère énantiomère pur.

12. Procédé selon le revendication 11, pour la préparation d'un composé selon l'une quelconque des revendications 2 à 4.

13. Composé selon l'une quelconque des revendications 1 à 4, pour utilisation en tant qu'agent thérapeutique.

14 Composé selon l'une quelconque des revendications 1 à 4, pour utilisation en tant qu'agent prophylactique dans le domaine médical.